# EUROPÄISCHE PATENTSCHRIFT

(11) **EP 0 956 876 B1**
(45) Veröffentlichungstag und Bekanntmachung des Hinweises auf die Patenterteilung: **09.06.2004**
(21) Anmeldenummer: 99105771.2
(22) Anmeldetag: 22.03.1999
(51) Int. Cl.: A61M 5/44

(54) **Kassette zur Förderung von Flüssigkeiten, insbesondere Dialyseflüssigkeiten**
Cassette for the delivery of fluids, especially dialysis fluids
Cassette pour le transport de liquides, spécialement de liquides de dialyse

(30) Priorität: 01.04.1998 DE 19814695
(43) Veröffentlichungstag der Anmeldung: 17.11.1999
(73) Patentinhaber: Fresenius Medical Care Deutschland GmbH, 61350 Bad Homburg v.d.H. (DE)
(72) Erfinder: Dönig, Rainer, 60489 Frankfurt (DE); Schulz, Wolfgang, 66606 St. Wendel (DE)
(74) Vertreter: Laufhütte, Dieter, Dr.-Ing.

(56) Entgegenhaltungen:
- EP-A- 0 482 858
- EP-A- 0 687 474
- EP-A- 0 778 033

## Beschreibung

Die vorliegende Erfindung betrifft eine Kassette zur Förderung von Flüssigkeiten, insbesondere Dialyseflüssigkeiten, mit Anschlußelementen zur Konnektion von Lösungsbeuteln und zum Patienten oder Dialysegerät führenden Leitungen, mit wenigstens einer Förderkammer mit Zu- und Ablauf sowie mit Leitungen zur Führung der zugeführten und geförderten Flüssigkeiten, wobei die Wandungen der Leitungen wenigstens abschnittsweise derart ausgeführt sind, daß die Leitungen durch das Aufbringen einer auf die Wandungen wirkenden Druckkraft verschließbar sind. Die vorliegende Erfindung betrifft femer ein Dialysegerät, in dem die Kassette zur Förderung von Flüssigkeiten, insbesondere Dialyseflüssigkeiten, aufnehmbar ist sowie ein Verfahren zum Fördern, Bilanzieren, Dosieren und Beheizen eines medizinischen Fluids.

Gattungsgemäße Kassetten zur Förderung von Flüssigkeiten, insbesondere Dialyseflüssigkeiten, sind aus der WO 97/09074 bekannt und werden üblicherweise als Austauschartikel in Dialysegeräten eingesetzt. Die Kassetten erfüllen hier die Aufgabe einer mediengetrennten Förderung der Dialyseflüssigkeit. Die in der Kassette angeordnete Förderkammer wird durch eine entsprechende Antriebseinheit des Dialysegerätes pneumatisch angetrieben. Auch ist der Einsatz mechanisch oder hydraulisch betriebener Förderkammem möglich. Die Förderkammer weist eine Membran auf, die durch die Antriebseinheit bewegt wird und die sicherstellt, daß eine Trennung zwischen den zu fördernden Medien und dem Arbeitsmedium des Dialysegerätes gewährleistet ist. Die Steuerung des Flüssigkeitsstromes in der Kassette erfolgt mittels pneumatisch betriebener Ventile. Um die dem Patienten zu verabreichende Dialyseflüssigkeit auf das gewünschte Temperatumiveau aufzuheizen und auf diesem Wert zu halten, sind in der WO 97/09074 Schalen offenbart, auf denen die Lösungsbeutel während der Dialyse gelagert werden. Die Schalen werden beispielsweise von Heizpatronen aufgeheizt, die ihrerseits von einer Steuereinheit des Dialysegerätes angesteuert werden.

Bei einer derartigen Anordnung ist es nachteilig, daß die Beheizung der Dialyseflüssigkeiten aufgrund der einseitigen Lagerung auf den Heizschalen ungleichmäßig erfolgt und mit erheblichen Wärmeverlusten verbunden ist. Femer ist eine separat angeordnete Heizstufe notwendig, was den Aufbau des Dialysegerätes entsprechend aufwendig gestaltet.

Eine gattungsgemäße Kassette zur Förderung von Flüssigkeiten, welche dem Oberbegriff von Anspruch 1 entspricht, ist in der EP-A-0 778 033 offenbart.

Es ist die Aufgabe der vorliegenden Erfindung, eine vereinfachte Vorrichtung zu schaffen, mit der die Heizung und Förderung von Flüssigkeiten, insbesondere von Dialyseflüssigkeiten, effektiv und einfach durchführbar ist.

In der Kassette ist wenigstens ein Bereich vorgesehen,in dem die Leitungen derart angeordnet sind, daß das in den Leitungen befindliche Medium durch eine außerhalb der Kassette angeordnete Heizvorrichtung auf einen vorgebbaren Sollwert aufheizbar ist. Die erfindungsgemäße Kassette weist, wie die Kassette aus der EP-A-0 778 033, die Vorteile auf, daß die Teilaufgaben fördern, dosieren/bilanzieren, mischen, messen, konnektieren und beheizen von medizinischen Fluiden in einer Einheit vereinigt bzw. gelöst werden können. Daraus ergibt sich eine wesentliche Erleichterung bei der Bedienung des Systems und eine Erhöhung der Fehlersicherheit aufgrund der Minimierung der risikobehafteten Arbeitsschritte. Die durchgängig mediengetrennte Ankopplung der erfindungsgemäßen Kassette erlaubt eine sterile Prozeßführung und schließt somit die Infektionsgefahr zuverlässig aus.

Gemäß der in Anspruch 1 definierten vorliegenden Erfindung umfaßt die Kassette (als charakterisierende Merkmale) einen festen Grundkörper sowie eine oder mehrere den Grundkörper wenigstens teilweise bedeckende Folien, die mit dem Grundkörper verbunden sind, wobei die Wandungen der Leitungen sowie der Förderkammer Unmittelbar durch den Grundkörper und die Folien gebildet werden. Im Bereich der Förderkammem dient die Folie bei entsprechend elastischer Ausführung als Membran, die durch eine Antriebseinheit, beispielsweise durch eine Kolbenpumpe, entsprechend der gewünschten Förderhöhe in der Förderkammer bewegt wird. Die Leitungen der Kassette, die zur Lenkung des Fluidstromes dienen, sind ebenfalls durch den Grundkörper und die diesen bedeckende Folie begrenzt. Dabei ist es möglich, daß nur eine Wandung der Leitungen oder auch mehrere Wandungen durch Folien gebildet werden.

In weiterer Ausgestaltung der vorliegenden Erfindung ist vorgesehen, daß sich die Folie auf beiden Seiten des Grundkörpers erstreckt. Dies ist insbesondere in dem Bereich der Kassette vorteilhaft, in dem die Leitungen derart angeordnet sind, daß das in den Leitungen befindliche Medium durch eine außerhalb der Kassette angeordnete Heizvorrichtung auf einen vorgebbaren Sollwert aufheizbar ist. In diesem Fall ist es möglich, entsprechend der beidseitigen Verwendung der Folie beide Seiten der Kassette und damit den entsprechend geführten Medienstrom effektiv zu beheizen.

Besonders vorteilhaft ist es, wenn eine Seite des Grundkörpers vollständig mit einer Folie bespannt ist. In diesem Fall dient die Folie im Bereich der Förderkammem als elastische Membran, die zum einen die Förderung beispielsweise der Dialyseflüssigkeit bewirkt und zum anderen eine Medientrennung ermöglicht. Im Bereich der Leitungen bildet die Folie eine der Wandungen und ermöglicht einen besonders günstigen und effektiven Wärmeübergang in das in der Leitung befindliche Medium.

Auch die Herstellung der Kassette wird in diesem Fall vereinfacht, da der Grundkörper lediglich Ausnehmungen oder Vertiefungen zur Bildung der Förderkammem oder Leitungen aufweist und anschließend erfindungsgemäß mit einer Folie bespannt wird, wodurch die Ausnehmungen zu Förderkammem und die in dem Grundkörper befindlichen Kanäle zu Leitungen vervollständigt werden. Die Folie kann verhältnismäßig einfach durch Verschweißen oder Verkleben auf den Grundkörper aufgebracht werden.

Gemäß einer bevorzugten Ausgestaltung der vorliegenden Erfindung sind zwei Förderkammem vorgesehen. Somit wird es möglich, bei wechselseitiger Betätigung der Förderkammem durch eine entsprechend abgestimmte Antriebseinheit einen verhältnismäßig gleichmäßigen Fluß durch die Kassette sowie ein angeschlossenes Dialysegerät bzw. Patienten zu erreichen. Ebenso ist es möglich, daß bei entsprechender Trennung der Förderkammem eine der Förderkammem für den Hochdruckbereich und eine andere beispielsweise für den Vakuum- oder Niederdruckbereich vorgesehen ist. Beide Förderkammem können durch den Grundkörper in der Kassette sowie durch die darauf aufgebrachte Folie begrenzt sein. Eine Steuerung des Medienstromes durch die Kassette bzw. eine entsprechende Ansteuerung der Förderkammem wird erfindungsgemäß dadurch erreicht, daß die Wandungen der Leitungen wenigstens abschnittsweise derart ausgeführt sind, daß die Leitungen durch das Aufbringen einer auf die Wandungen wirkenden Druckkraft verschließbar sind. Diese Druckkraft kann beispielsweise pneumatisch oder auch mechanisch durch Ventilstößel aufgebracht werden.

In weiterer Ausgestaltung der vorliegenden Erfindung ist vorgesehen, daß die Leitungen wenigstens in dem beheizbaren Bereich der Kassette spiralförmig angeordnet sind. Daraus ergibt sich der Vorteil, daß eine verhältnismäßig große Leitungslänge auf einer verhältnismäßig geringen Fläche der Kassette angeordnet ist, was eine besonders effektive Beheizung der in den Leitungen befindlichen Medien ermöglicht. Neben einer spiralförmigen Anordnung sind weitere Ausgestaltungen denkbar, die stets dann besonders effektiv sind, wenn das Verhältnis von Leitungslänge zu Flächenbedarf der Leitungen große Werte annimmt, ebenso wie bei Erreichen einer turbulenten Strömung auch schon bei kleineren Flußraten.

Besonders vorteilhaft ist es, wenn sich auf beiden Seiten des Grundkörpers Bereiche mit spiralförmig angeordneten Leitungen erstrecken. Dadurch wird es möglich, das zu heizende Fluid zunächst auf einer Seite der Kassette mit einer entsprechend angeordneten Heizvorrichtung aufzuheizen und dieses beim Passieren der anderen Seite der Kassette auf der gewünschten Temperatur zu halten oder je nach Bedarf weiter aufzuheizen.

Die auf unterschiedlichen Seiten des Grundkörpers befindlichen Bereiche können mit einer im Grundkörper angeordneten Bohrung verbunden sein. Somit ist es möglich, die Medien zunächst auf einer Seite des Grundkörpers zu führen und zu beheizen und anschließend durch eine Bohrung auf die andere Seite zu leiten und hier weiter aufzuheizen, was besonders effektiv ist, wenn die Leitungen auf beiden Seiten spiralförmig angeordnet sind.

Gemäß einer bevorzugten Ausgestaltung der vorliegenden Erfindung besteht der Grundkörper wenigstens teilweise aus Kunststoff. Die Schaffung des Grundkörpers aus Kunststoff weist den Vorteil auf, daß dieser beispielsweise durch Spritzgußverfahren besonders einfach und in besonders vielen denkbaren Ausgestaltungen herstellbar ist.

Besonders vorteilhaft ist es, wenn der Grundkörper Halterungen zur Montage von Meßwertaufnehmem aufweist. Insbesondere können Halterungen zur Montage von Sensoren für Temperatur und Druck sowie Flußrate an dem Grundkörper angeordnet sein. Beispielsweise können die Temperatursensoren durch die Folie hindurch die Temperatur der Dialyselösung erfassen und die Werte einer Auswerteeinheit übergeben. Zur Regelung eines Temperatursollwertes können entweder die Fördergeschwindigkeit durch die beheizten Leitungen oder die Heizleistung bei konstanter Fördergeschwindigkeit verändert werden.

Gemäß einer bevorzugten Ausgestaltung der vorliegenden Erfindung ist eine zum Patienten führende Leitung und ein Drainageschlauch vorgesehen, die unlösbar mit der Kassette verbunden sind. Somit wird die Kassette mit diesen Leitungen hergestellt, während die Belegung der weiteren Anschlußelemente der Kassette entsprechend dem geforderten Dialyseverfahren vom Bediener gewählt und durchgeführt wird.

Die Erfindung betrifft femer ein Dialysegerät, insbesondere zur Durchführung von Peritoneal-Dialyse-, Hemo- und Plasma-Filtrationsverfahren, mit einer Aussparung oder Vorrichtung in der eine Kassette nach einem der Ansprüche 1-10 aufgenommen ist, sowie mit einer Pumpeinheit zur Betätigung der Förderkammem der Kassette. Zur Beheizung der erfindungsgemäßen Kassette ist eine Heizvorrichtung vorgesehen, die im Bereich der Aussparung oder Vorrichtung zur Aufnahme der Kassette angeordnet ist. Erfindungsgemäß ist es somit möglich, bei jedem Patienten stets eine neue Kassette zu verwenden, die in das Dialysegerät eingeschoben oder daran in geeigneter Weise angeordnet wird. Die mediengetrennte Ankopplung der Kassette an das Dialysegerät ermöglicht eine sterile Prozeßführung und stellt aseptische Bedingungen auch dann sicher, wenn das Dialysegerät von mehreren Patienten benutzt wird. Eine Infektionsgefahr ist wirksam dadurch ausgeschlossen, daß die Kassette vorteilhaft als Disposable ausgeführt ist und nach der Verwendung nicht wieder eingesetzt wird.

Insgesamt ergibt sich ein einfaches zuverlässiges und wirksames Dialysesystem, bei dem der Bediener nach Eingabe der gewünschten Sollwerte lediglich die Kassette in die dafür vorgesehene Aussparung oder in der dafür vorgesehenen Vorrichtung anordnen und die notwendigen Leitungen konnektieren muß. Eine separate Anordnung von Heizvorrichtungen oder -elementen ist nicht notwendig.

In weiterer Ausgestaltung der vorliegenden Erfindung ist vorgesehen, daß die Heizvorrichtung Flächenheizelemente aufweist. Dies ist insbesondere dann von Vorteil, wenn die Kassette flächige Bereiche aufweist, die beispielsweise spiralförmig angeordnete Leitungen enthalten. In diesem Fall ist eine besonders effektive Beheizung der durch die Leitungen strömenden Medien möglich.

Die Heizvorrichtung kann sich auf beiden Seiten der Aussparung oder Vorrichtung zur Aufnahme der Kassette derart erstrecken, daß die Kassette beidseitig beheizbar ist. In diesem Falle ergibt sich bei verhältnismäßig geringem Platzbedarf auch bei hohen Flußraten eine wirksame Beheizung der Medien auf den gewünschten **Sollwert.**

Ferner wird an dieser Stelle ein Verfahren zum Fördem, Bilanzieren, Dosieren und Beheizen eines medizinischen Fluids offenbart, bei dem ein durch Leitungen und wenigstens eine Förderkammer gefördertes Fluid gleichzeitig mit Heizenergie beaufschlagt wird. Daraus ergibt sich ein einfach durchzuführendes Verfahren, bei dem beispielsweise die Teilaufgaben Fördern, Dosieren/Bilanzieren, Mischen, Messen, Konnektieren und Beheizen von medizinischen Fluiden in kompakter Weise kombiniert bzw. gelöst werden können.

Besonders vorteilhaft ist es, wenn das Fluid zunächst durch die Förderkammem geführt wird und anschließend in einem Leitungen umfassenden Bereich beheizt wird. Ebenso ist es jedoch möglich, daß die Beheizung bereits oder ausschließlich in den Förderkammern erfolgt. Die Beheizung des Fluids in Leitungen weist den Vorteil auf, daß durch eine günstige Anordnung der Leitungen auf verhältnismäßig geringer Fläche eine große Leitungslänge angeordnet werden kann. Dadurch ergibt sich die Möglichkeit, das Fluid auch bei hohen Durchflußraten gezielt und wirksam zu beheizen.

Die Beheizung kann in einem Bereich erfolgen, in dem die Leitungen spiralförmig angeordnet sind. Hierbei ergibt sich ein besonders günstiges Verhältnis aus Leitungslänge pro Flächeneinheit, womit eine besonders effektive Beheizung möglich ist.

In weiterer Ausgestaltung der Erfindung ist vorgesehen, daß das Fluid zunächst durch die wenigstens eine Förderkammer einer für die Förderung, Bilanzierung, Dosierung und Beheizung des Fluids vorgesehenen Kassette geführt und in auf einer Seite der Kassette befindlichen Leitungen beheizt wird, wonach eine Überführung des Fluids auf die andere Seite der Kassette und eine weitere Beheizung in auf dieser Seite befindlichen Leitungen erfolgt. Bei einer derartigen Anordnung ergibt sich aufgrund der verhältnismäßig großen sich auf beiden Seiten der Kassette erstreckenden Leitungslänge die Möglichkeit, auch bei hohen Durchflußraten eine ausreichende Beheizung des Fluids zu bewirken.

Weitere Vorteile und Einzelheiten der vorliegenden Erfindung werden anhand eines in der Zeichnung dargestellten Ausführungsbeispiels näher erläutert. Es zeigt:

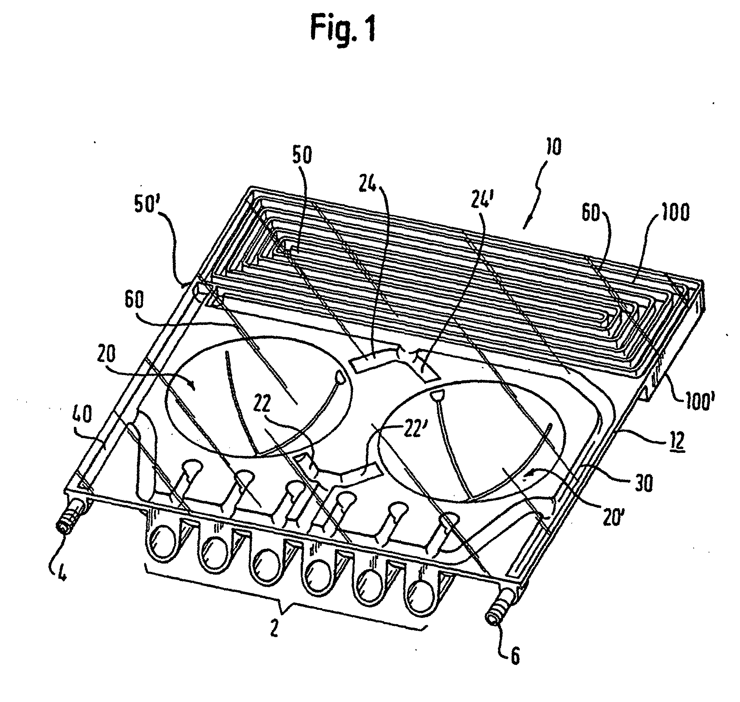

- Fig. 1:: eine perspektivische Darstellung einer erfindungsgemäßen Kassette mit zwei Förderkammem sowie mit Bereichen zur Beheizung der geförderten Fluide.

Fig. 1 zeigt die erfindungsgemäße Kassette 10, die als Disposable ausgeführt ist und die in eine entsprechend gestalteten Aussparung oder Aufnahme eines Dialysegerätes eingesteckt werden kann. Die Bezugszeichen 2, 4, 6 kennzeichnen Anschlußelemente zur Konnektion von Lösungsbeuteln, zum Patienten oder Dialysegerät führenden Leitungen oder auch Drainageleitungen. An den Anschlüssen 4 und 6 sind zwei (nicht dargestellte) fest angebrachte Schläuche angeordnet, von denen einer den Patientenschlauch und der andere den Drainageschlauch darstellt. Die Anschlußelemente 2 dienen der durch den Bediener durchzuführenden Konnektion, beispielsweise von Lösungsbeuteln oder anderen Medikamentenbehältem.

Die Kassette 10 umfaßt den Grundkörper 12 der aus Kunststoff besteht und in Spritzgußtechnik oder auch Tiefziehtechnik herstellbar ist. In dem Grundkörper 12 erstrecken sich Ausnehmungen sowie Kanäle, die zum Teil die Wandungen der zwei nebeneinander angeordneten Förderkammern 20, 20' sowie der in die Kassette angeordneten Leitungen 30, 40, 50, 50' bilden. Auch die von den Anschlußelementen 2 ausgehenden Leitungen sowie die Zuläufe 22, 22' und Abläufe 24, 24' der Förderkammern 20, 20' werden teilweise durch den Grundkörper 12 gebildet.

Über der in Fig. 1 oben dargestellten Seite des Grundkörpers 12 erstreckt sich über die gesamte Fläche eine Folie 60, die mit dem Grundkörper 12 verschweißt ist. Die Folie 60 bildet die Begrenzungen der im Grundkörper 12 gebildeten Kanäle und Ausnehmungen, wodurch beispielsweise die Leitungen 30, 40, 50, 50' sowie die Förderkammem 20, 20' begrenzt werden. Auch die Zuläufe 22, 22' und Abläufe 24, 24' der Förderkammern 20, 20' werden auf einer Seite durch die Folie 60 begrenzt.

In dem gemäß Fig. 1 oberhalb der Förderkammem 20, 20' angeordneten Bereichen 100, 100' sind die Leitungen 50, 50' derart angeordnet, daß das in den Leitungen 50, 50' befindliche Medium durch eine außerhalb der Kassette 10 angeordnete Heizvorrichtung auf einen vorgebbaren Sollwert aufheizbar ist. Dabei erstrecken sich die Leitungen 50, 50' sowohl auf der in Fig. 1 dargestellten Oberseite (Leitungen 50) sowie auf der Unterseite (Leitungen 50').

Die Steuerung der zugeführten bzw. geförderten Medien erfolgt derart, daß die Wandungen beispietsweise der Leitungen 30, 40, 50, 50' sowie des Zulaufes 22, 22' und des Ablaufes 24, 24' der Förderkammem 20, 20' durch das Aufbringen einer auf die Wandungen wirkenden Druckkraft verschließbar sind. Diese Druckkraft kann beispielsweise pneumatisch, hydraulisch oder auch mechanisch durch Ventilstößel aufgebracht werden, die von einer Steuereinheit des Dialysegerätes angesteuert werden können.

Der Anschluß von Lösungsbeuteln und weiteren Medikamentenbehältern erfolgt Ober die in Fig. 1 mit 2 bezeichneten Anschlußelemente, die zusätzlich über einen Berührschutz verfügen. Die zugeführten Medien werden bei entsprechender Ventilstellung mittels der Zuläufe 22, 22' in die Förderkammern 20 bzw. 20' eingeführt und durch die durch die Antriebseinheit des Dialysegerätes bewirkte Bewegung der Folie 60 aus den Förderkammern 20, 20' herausgepumpt. Dies geschieht mittels der Abläufe 24, 24', die zusammentreffen und in einen ersten Abschnitt der Leitung 50 münden. Das geförderte Medium durchströmt nun die auf der Oberseite im Bereich 100 befindliche Leitung 50 und wird dabei durch eine entsprechend angeordnete Heizvorrichtung des Dialysegerätes aufgeheizt. Im Endbereich der spiralförmig angeordneten Leitung 50 befindet sich eine (nicht dargestellte) Bohrung, die eine Verbindung zum auf der Unterseite befindlichen Bereich 100' des Grundkörpers 12 bildet. Entsprechend strömt das geförderte Medium durch diese Bohrung auf die andere Seite des Grundkörpers 12 in die ebenfalls spiralförmig angeordnete Leitung 50', wobei wiederum eine Beheizung des Fluids stattfinden kann. Im Anschluß daran verläßt die beheizte Flüssigkeit über den Konnektor 4 bzw. den daran angeschlossenen Schlauch die erfindungsgemäße Kassette 10 zum Patienten bzw. zum Dialysegerät.

Die erfindungsgemäße Kassette und das erfindungsgemäße Dialysegerät eignen sich für die Verwendung bei der Peritoneal-Dialyse sowie bei der Hemo- und Plasma-Filtration. Entsprechend ist der Einsatz bei Peritoneal-Systemen und Systemen mit extrakorporalem Blutkreislauf möglich.

Die Geometrie der Leitungen gemäß Fig. 1 erlaubt eine einfache Steuerung der Medien durch Verschließen der Leitungen mittels der Ankopplung beispielsweise von Ventilstößeln, wodurch eine einwandfreie Funktion im Über- wie auch im Unterdruckbereich möglich ist. Die Beheizung der Bereiche 100, 100' erfolgt entsprechend vorder- und rückseitig mittels (z.B. ohmscher) Flächenheizelementen. Die Geometrie der Leitungen 50, 50' ist speziell für einen guten Wärmeübergang, bei gleichzeitig niedrigen Drosselverlusten, optimiert. Trotz hoher Flächenleistungsdichte (z.B. ca. 5 W/cm²) bleiben die Oberflächentemperaturen der Heizelemente niedrig (< 100° C). Dies hält die Anforderungen an die Temperaturstabilität der Folie 60 und des Grundkörpers 12 gering.

Die Leitungen 40 und 30 sind beispielsweise für frische Dialyselösungen bzw. für verbrauchtes Dialysat in der automatischen Peritoneal-Dialyse einsetzbar.

Durch die Kombination von mediengetrennten Fördereigenschaften sowie der günstigen Heizeigenschaften ist die erfindungsgemäße Kassette bzw. das Dialysegerät vorteilhaft bei der Automatischen Peritoneal-Dialyse (APD) sowie auch bei diversen Hemo- und Plasma-Filtrationsverfahren (Universal HDF, CWHF, CWHDF, PF etc.) sowie bei Adsorberanwendungen in Kombination mit einem extrakorporalen Blutkreislauf einsetzbar.

## Patentansprüche

1. Kassette (10) zur Förderung von Flüssigkeiten, insbesondere Dialyseflüssigkeiten,
mit Anschlußelementen (2,4,6) zur Konnektion von Lösungsbeuteln und zum Patienten oder Dialysegerät führenden Leitungen,
mit wenigstens einer Förderkammer (20,20') mit Zu- und Ablauf (22,22',24,24'),
mit Leitungen (30,40,50,50') zur Führung der zugeführten und geförderten Flüssigkeiten,
wobei die Wandungen der Leitungen (30,40,50,50') wenigstens abschnittsweise derart ausgeführt sind, daß die Leitungen (30,40,50,50') durch das Aufbringen einer auf die Wandungen wirkenden Druckkraft verschließbar sind
wobei in der Kassette (10) wenigstens ein Bereich vorgesehen ist, in dem die Leitungen (50, 50') derart angeordnet sind, dass pro Flächeneinheit der Kassette (10) eine größere Leitungslänge vorliegt als in anderen Bereichen der Kassette (10), und in dem die in den Leitungen (50,50') befindliche Flüssigkeit durch eine außerhalb der Kassette (10) angeordnete Heizvorrichtung aufheizbar ist,
**dadurch gekennzeichnet,**
**dass** die Kassette (10) einen festen Grundkörper (12) sowie eine oder mehrere den Grundkörper (12) wenigstens teilweise bedeckende Folien (60) umfaßt, die mit dem Grundkörper (12) verbunden sind, wobei die Wandungen der Leitungen (30,40,50,50') sowie der Förderkammer (20,20') einerseits unmittelbar durch den Grundkörper (12) und andererseits unmittelbar durch die Folien (60) gebildet werden.

2. Kassette (10) nach Anspruch 2, **dadurch gekennzeichnet, daß** sich die Folie (60) auf beiden Seiten des Grundkörpers (12) erstreckt.

3. Kassette (10) nach Anspruch 1 oder 2, **dadurch gekennzeichnet, daß** eine Seite des Grundkörpers (12) vollständig mit einer Folie (60) bespannt ist.

4. Kassette (10) nach einem der Ansprüche 1 bis 3, **dadurch gekennzeichnet, daß** zwei Förderkammem (20,20') vorgesehen sind.

5. Kassette (10) nach einem der Ansprüche 1 bis 4, **dadurch gekennzeichnet, daß** die Leitungen (50,50') wenigstens in dem beheizbaren Bereich (100,100') der Kassette (10) spiralförmig angeordnet sind.

6. Kassette (10) nach Anspruch 5, **dadurch gekennzeichnet, daß** sich auf beiden Seiten des Grundkörpers (12) Bereiche (100, 100') mit spiralförmig angeordneten Leitungen erstrecken.

7. Kassette (10) nach Anspruch 6, **dadurch gekennzeichnet, daß** die auf unterschiedlichen Seiten des Grundkörpers (12) befindlichen Bereiche (100,100') mit einer im Grundkörper (12) angeordneten Bohrung verbunden sind.

8. Kassette (10) nach einem der Ansprüche 1 bis 7, **dadurch gekennzeichnet, daß** der Grundkörper (12) wenigstens teilweise aus Kunststoff besteht.

9. Kassette (10) nach einem der Ansprüche 1 bis 8, **dadurch gekennzeichnet, daß** der Grundkörper (12) Halterungen zur Montage von Meßwertaufnehmem aufweist.

10. Kassette (10) nach einem der Ansprüche 1 bis 9, **dadurch gegekennzeichnet**, daß eine zum Patienten führende Leitung und ein Drainageschlauch vorgesehen sind, die unlösbar mit der Kassette (10) verbunden sind.

11. Dialysegerät, insbesondere zur Durchführung von Peritoneal-Dialyse-, Hemound Plasma-Filtrationsverfahren, mit einer Aussparung oder Vorrichtung, in der eine Kassette (10) nach einem der Ansprüche 1 bis 10 aufgenommen ist, mit einer Pumpeinheit zur Betätigung der Förderkammem (20,20') der Kassette (10) sowie mit einer Heizvorrichtung, die im Bereich der Aussparung oder Vorrichtung zur Aufnahme der Kassette (10) angeordnet ist.

12. Dialysegerät nach Anspruch 11, **dadurch gekennzeichnet, daß** die Heizvorrichtung Flächenheizelemente aufweist.

13. Dialysegerät nach Anspruch 11 oder 12, **dadurch gekennzeichnet, daß** sich die Heizvorrichtung auf beiden Seiten der Aussparung oder Vorrichtung zur Aufnahme der Kassette (10) derart erstreckt, daß die Kassette (10) beidseitig beheizbar ist.

## Claims

1. A cassette (10) for the delivery of fluids, in particular dialysis fluids, comprising
connecting elements (2, 4, 6) for the connection of solution bags and conduits leading to the patient or dialysis apparatus,
at least one delivery chamber (20, 20') with a feed and a discharge (22, 22', 24, 24'),
conduits (30, 40, 50, 50') for guiding the fed and delivered fluids,
wherein the walls of the conduits (30, 40, 50, 50') are at least in portion-wise manner so designed that the conduits (30, 40, 50, 50') are closable by the application of a pressure force acting on the walls,
wherein provided in the cassette (10) is at least one region in which the conduits (50, 50') are so arranged that per unit of area of the cassette (10) there is a greater conduit length than in other regions of the cassette (10) and in which the fluid in the conduits (50, 50') is heatable by a heating device arranged outside the cassette (10),
**characterised in that**
the cassette (10) includes a sturdy main body (12) and one or more films (60) which at least partially cover the main body (12) and which are connected to the main body (12), the walls of the conduits (30, 40, 50, 50') and of the delivery chamber (20, 20') being formed on the one hand directly by the main body (12) and on the other hand directly by the films (60).

2. A cassette (10) according to claim 2 **characterised in that** the film (60) extends on both sides of the main body (12).

3. A cassette (10) according to claim 1 or claim 2 **characterised in that** one side of the main body (12) is completely covered with a film (60).

4. A cassette (10) according to one of claims 1 to 3 **characterised in that** there are provided two delivery chambers (20, 20').

5. A cassette (10) according to one of claims 1 to 4 **characterised in that** the conduits (5, 50') are arranged in a spiral form at least in the heatable region (100, 100') of the cassette (10).

6. A cassette (10) according to claim 5 **characterised in that** regions (100, 100') with conduits arranged in a spiral form extend on both sides of the main body (12).

7. A cassette (10) according to claim 6 **characterised in that** the regions (100, 100') disposed on different sides of the main body (12) are connected to a bore arranged in the main body (12).

8. A cassette (10) according to one of claims 1 to 7 **characterised in that** the main body (12) at least partially comprises plastic material.

9. A cassette (10) according to one of claims 1 to 8 **characterised in that** the main body (12) has holders for mounting measurement value pick-up devices.

10. A cassette (10) according to one of claims 1 to 9 **characterised in that** there are provided a conduit leading to the patient and a drainage hose, which are non-releasably connected to the cassette (10).

11. A dialysis apparatus, in particular for carrying out peritoneal dialysis, haemo- and plasma-filtration processes, comprising an opening or device in which a cassette (10) according to one of claims 1 to 10 is accommodated, comprising a pump unit for actuation of the delivery chambers (20, 20') of the cassette (10) and a heating device which is arranged in the region of the opening or device for accommodating the cassette (10).

12. A dialysis apparatus according to claim 11 **characterised in that** the heating device has surface heating elements.

13. A dialysis apparatus according to claim 11 or claim 12 **characterised in that** the heating device extends on both sides of the opening or device for accommodating the cassette (10) in such a way that the cassette (10) is heatable on both sides.

## Revendications

1. Cassette (10) pour le transport de liquides, en particulier de liquides de dialyse,
avec des éléments de raccordement (2, 4, 6) pour la connexion de sachets de solution et de conduites allant au patient ou à l'appareil de dialyse,
avec au moins une chambre de transport (20, 20') avec arrivée et sortie (22, 22', 24, 24'),
avec des conduites (30, 40, 50, 50') pour le guidage des liquides amenés et transportés,
les parois des conduites (30, 40, 50, 50') étant réalisées au moins par zones de telle sorte que les conduites (30, 40, 50, 50') peuvent être fermées en appliquant une force de pression agissant sur les parois
au moins une zone étant prévue dans la cassette (10), dans laquelle les conduites (50, 50') sont disposées de telle sorte que, par unité de surface de la cassette (10), on a une longueur de conduite plus grande que dans d'autres zones de la cassette (10), et dans laquelle le liquide se trouvant dans les conduites (50, 50') peut être réchauffé par un dispositif de chauffage disposé à l'extérieur de la cassette (10),
**caractérisée en ce que**
la cassette (10) comprend un corps de base (12) fixe ainsi qu'une ou plusieurs feuilles (60) recouvrant au moins partiellement le corps de base (12), qui sont reliées au corps de base (12), les parois des conduites (30, 40, 50, 50') et de la chambre de transport (20, 20') étant formées d'une part directement par le corps de base (12) et d'autre part directement par les feuilles (60).

2. Cassette (10) selon la revendication 2, **caractérisée en ce que** la feuille (60) s'étend sur les deux côtés du corps de base (12).

3. Cassette (la) selon la revendication 1 ou 2, **caractérisée en ce qu'**un côté du corps de base (12) est tendu complètement par une feuille (60).

4. Cassette (10) selon l'une quelconque des revendications 1 à 3, **caractérisée en ce que** deux chambres de transport (20, 20') sont prévues.

5. Cassette selon l'une quelconque des revendications 1 à 4, **caractérisée en ce que** les conduites (50, 50') sont disposées en forme de spirale au moins dans la zone (100, 100') pouvant être chauffée de la cassette (10).

6. Cassette (10) selon la revendication 5, **caractérisée en ce que** des zones (100, 100') avec des conduites disposées en forme de spirale s'étendent sur les deux côtés du corps de base (12).

7. Cassette (10) selon la revendication 6, **caractérisée en ce que** les zones (100, 100') se trouvant sur différents corps de base (12) sont reliées à un perçage disposé dans le corps de base (12).

8. Cassette (10) selon l'une quelconque des revendications 1 à 7, **caractérisée en ce que** le corps de base (12) est au moins en partie à base de matière synthétique.

9. Cassette (10) selon l'une quelconque des revendications 1 à 8, **caractérisée en ce que** le corps de base (12) présente des fixations pour le montage d'enregistreurs de valeurs de mesure.

10. Cassette (10) selon l'une quelconque des revendications 1 à 9, **caractérisée en ce qu'**une conduite allant vers le patient et un flexible de drainage sont prévus, lesquels sont reliés de façon indétachable à la cassette (10).

11. Appareil de dialyse, en particulier pour l'application de procédés de dialyse péritonéale, d'hémofiltration et de filtration à plasma, avec un évidement ou un dispositif, dans lequel est réceptionnée une cassette (10) selon l'une quelconque des revendications 1 à 10, avec une unité de pompage pour l'actionnement des chambres de transport (20, 20') de la cassette (10) et avec un dispositif de chauffage, qui est disposé dans la zone de l'évidement ou du dispositif pour le logement de la cassette (10).

12. Appareil de dialyse selon la revendication 11, **caractérisé en ce que** le dispositif de chauffage présente des éléments de chauffage de surface.

13. Appareil de dialyse selon la revendication 11 ou 12, **caractérisé en ce que** le dispositif de chauffage s'étend sur les deux côtés de l'évidement ou du dispositif pour le logement de la cassette (10) de telle sorte que la cassette (10) peut être chauffée des deux côtés.
